# EUROPEAN PATENT SPECIFICATION

(11) **EP 3 405 135 B1**
(45) Date of publication and mention of the grant of the patent: **11.03.2020**
(21) Application number: 17704529.1
(22) Date of filing: 02.02.2017
(51) Int. Cl.: A61C 1/08

(54) **DENTAL IMPLANT DRILL-GUIDE SYSTEM**
FÜHRUNGSSYSTEM FÜR ZAHNIMPLANTATBOHRER
SYSTÈME DE GUIDAGE POUR FORET D'IMPLANT DENTAIRE

(30) Priority: 13.02.2016 GB 201602598; 15.08.2016 GB 201613924
(43) Date of publication of application: 28.11.2018
(73) Proprietor: Nulty, Adam Brian, Bury, Lancashire BL9 0NT (GB)
(72) Inventor: Nulty, Adam Brian, Bury, Lancashire BL9 0NT (GB)
(74) Representative: Doherty, William
(86) International application number: PCT/GB2017/050251
(87) International publication number: WO 2017/137724

(56) References cited:
- EP-A1- 2 103 276
- WO-A1-03/003933
- KR-A- 20110 055 907
- US-A1- 2007 077 535
- US-A1- 2011 045 431
- US-A1- 2012 010 740

## Description

The present invention relates to a dental implant drill-guide system.

A method of creating a dental implant drill-guide, in particular for the drilling of dental implant cavities in an edentulous maxillary or mandibular arch of a patient, is disclosed herein. The disclosure further relates to a method of assembling a drill guide for the insertion of dental implants, and to a method of implanting one or more dental implants into a patient.

Dental implants are artificial replacements for missing teeth which interface with the bone of the maxilla or mandible. Often the term dental implant refers solely to the bone-interfacing element, which is often screw threaded, to which a false crown of a tooth is mounted.

Ordinarily, if only a single tooth or a few teeth are missing, then the dental surgeon is able to align the dental implant to the remaining teeth. However, where a patient is fully edentulous, that is, missing all teeth emanating from the mandible or maxilla, then there is a difficulty in referencing the positions in which the dental implants should be inserted.

One option is to cut through the gingival mucosa or gums to expose the underlying bone structure, allowing the dental surgeon to determine visually where to drill to insert the dental implant. This is highly traumatic to the patient, and can take a significant amount of time for the mucosa to heal.

An alternative is to take a mucosal impression using an impression body, imaging the osseous tissue underneath the gingival mucosa, and then applying a drill guide over the mucosa to align the dental drill bit. The problem with this approach is that when the mucosal impression is taken, the gingival mucosa deform or compress under the force applied, skewing the information available to the dental surgeon. This can result in misaligned dental implants, even where a drill guide is used.

EP 2103276 A1, US 2012/0010740 A1 and WO 03/003933 A1 disclose examples of a dental implant drill-guide system comprising a dental implant drill-guide and a plurality of two-part orthodontic screws.

The present disclosure seeks to provide a method of forming a drill guide which is more accurate than that currently available, without significantly increasing the need for invasive surgery at or adjacent to the gums of the patient.

According to an aspect not in accordance with the invention, there is provided a method of creating a dental implant drill-guide for guiding the drilling of dental-implant cavities in an edentulous maxillary or mandibular arch, the method comprising the steps of: a] using mucosal impression data of an edentulous maxillary or mandibular arch having a plurality of orthodontic screws inserted into the osseous tissue thereof and which extend through the gingival mucosa, along with image data of the edentulous maxillary or mandibular arch to determine dental implant position data for the insertion of one or more dental implants into the edentulous maxillary or mandibular arch relative to the positions of the plurality of orthodontic screws; b] forming dental implant drill-guide parameter data from the determined dental implant position data; and c] creating a dental implant drill-guide from the determined dental implant drill-guide parameter data, the dental implant drill-guide having a plurality of orthodontic screw-seats which correspond to the said plurality of orthodontic screws of the mucosal impression data.

By relying on data relating to orthodontic screws inserted into the maxillary or mandibular arch prior to imaging the jaw, only a small amount of penetration of the gingival mucosa is required. However, the orthodontic screws are able to anchor into the mandible or maxilla of the patient, providing an accurate and reproducible reference frame for the operator when manufacturing a bespoke drill guide for the patient. This ensures that the drill guide is correctly aligned with respect to the bone, rather than the mucosal tissue, which is compressible and mobile.

Preferably, during step a], the mucosal impression data may be determined using three said orthodontic screws which are inserted in a triangular arrangement in the edentulous maxillary or mandibular arch. Optionally, during step a], the mucosal impression data may be determined using the plurality of orthodontic screws which are inserted between 2 and 3 millimetres deep into the osseous tissue of the edentulous maxillary or mandibular arch. During step a], a digital mucosal impression of the edentulous maxillary or mandibular arch may be obtained.

A triangulated arrangement of orthodontic screws has the advantage of providing sufficient structural support to the dental implant drill guide after seating onto the screws, whilst minimising the need to invasively insert the screws through the gingival mucosa of the patient. Minimisation of the pain caused to the patient is therefore of prime concern.

The method may further comprise a step prior to step a] of obtaining pre-operative imaging data of the edentulous maxillary or mandibular arch to determine an optimal position for the plurality of orthodontic screws and/or an optimal position of the dental-implant cavities. Said pre-operative imaging data is radiographic imaging data. Preferably, during step a], the imaging data may be Cone Beam Computed Tomography imaging data.

By imaging the bone structure of the patient prior to insertion of the orthodontic screws, the major nerves can be readily avoided, and a most effective arrangement of dental implant cavities with respect to orthodontic screw positions can be ascertained.

Optionally, during step c], the method may further comprise the step of engaging the orthodontic screw seats of the dental implant with the plurality of orthodontic screws using a positive locator. The positive locators may preferably include a collar of each orthodontic screw which is receivable by the dental implant drill-guide
The better the engagement between the drill guide and the orthodontic screws, the simpler it will be to secure the drill guide into position, advantageously simplifying the installation process for the dental surgeon.

Preferably, the apical part of the two-part orthodontic screw is a non-integrating orthodontic screw, the apical part of the two-part orthodontic screw acts as a positional reference for the dental implant drill-guide.

According to the present invention, there is provided a dental implant drill-guide system according to claim 1. Further embodiments of the invention are defined in the dependent claims.

The provision of the two-part orthodontic screws allows for a simple and effective means of clamping a dental guide in position, whilst the orthodontic screw seats on the dental implant drill guide advantageously simplifies the insertion and engagement of the drill guide in situ.

Preferably, the or each second aperture may be larger than the or each first aperture of the dental implant drill-guide.

Not only can the size of the apertures be made so as to minimise spatial constraints whilst providing sufficient drilling and screwing room, but this also acts as a straightforward visual guide for a dental surgeon to assist with alignment and fixation of the drill guide into position.

Optionally, the guide body may comprise an anterior frame element, a posterior frame element, and at least one bridging elements, the first and second apertures being located within the or each bridging element, and/or at least one buccal abutment element.

The guide body is beneficially formed so as to be readily receivable about the edentulous maxillary or mandibular arch of a patient, whilst also providing ample space for a dental surgeon to be able to operate within the restricted oral cavity.

Preferably, the coronal part of each two-part orthodontic screw may be held captive within each respective first aperture.

By holding the coronal part of each orthodontic screw with respect to the guide body, the dental surgeon can safely manipulate and engage the screw without there being a risk of the coronal part of the screw becoming dislodged and entering into the patient's oesophagus or trachea. Furthermore, if held captive within the drill-guide, the risk of losing the orthodontic screws in transit is also minimised.

According to an aspect not in accordance with the invention, there is provided a method of creating a dental implant drill-guide, the method comprising the steps of: a] using three-dimensional oral information data of an edentulous maxillary or mandibular arch having a plurality of orthodontic screws inserted into the osseous tissue thereof and which extend through the gingival mucosa to determine dental implant position data for the insertion of one or more dental implants into the edentulous maxillary or mandibular arch relative to the positions of the plurality of orthodontic screws; b] forming dental implant drill-guide parameter data from the determined dental implant position data; and c] creating a dental implant drill-guide from the determined dental implant drill-guide parameter data, the dental implant drill-guide having a plurality of orthodontic screw-seats which correspond to the said plurality of orthodontic screws of the three-dimensional impression data.

It will be apparent that whilst current techniques for determining oral geometry require a two-stage information gathering process, that is, the imaging of the jaw and the taking of an oral impression, it may be possible to fully reconstruct the oral geometry in a single stage, in which case, the present methodology will still be applicable.

According to an aspect not in accordance with the invention, there is provided a method of assembling a drill guide for the insertion of dental implants, the method comprising the steps of: a] providing a dental implant drill-guide system, preferably in accordance with the third aspect of the invention; b] engaging the orthodontic screw-seats of the dental implant drill-guide respectively with the apical parts of the two-part orthodontic screws; and c] fixing the dental implant drill-guide in position by engaging the coronal parts of the two-part orthodontic screws with the apical parts of the two-part orthodontic screws so as to clamp the dental implant drill-guide into position.
According to an aspect not in accordance with the invention, there is provided a method of implanting one or more dental implants into a patient, the method comprising the steps of: a] inserting a plurality of apical parts of two-part orthodontic screws into an edentulous maxillary or mandibular arch of a patient, such that the two-part orthodontic screws engage with the osseous tissue of the edentulous maxillary or mandibular arch; b] obtaining a mucosal impression of the edentulous maxillary or mandibular arch; c] imaging the maxilla or mandible of the patient inclusive of the plurality of two-part orthodontic screws to form a maxillary or mandibular image; d] determining from the maxillary or mandibular image and the mucosal impression one or more dental implant positions for the insertion of dental implants into the edentulous maxillary or mandibular arch relative to the positions of the plurality of two-part orthodontic screws; e] creating a dental implant drill-guide based on the one or more dental implant positions and the positions of the plurality of two-part orthodontic screws; f] positioning the dental drill-guide on the apical parts of the two-part orthodontic screws; g] fixing the dental implant drill-guide in position by engaging the coronal parts of the two-part orthodontic screws with the apical parts of the two-part orthodontic screws so as to clamp the dental implant drill-guide; h] drilling one or more dental-implant cavities in the edentulous maxillary or mandibular arch using the dental implant drill-guide; and i] inserting a dental implant into each dental-implant cavity.
Preferably, during step i], the dental implants may be guided into the respective dental-implant cavities by the dental implant drill-guide
The invention will now be more particularly described, by way of example only, with reference to the accompanying drawings, in which:

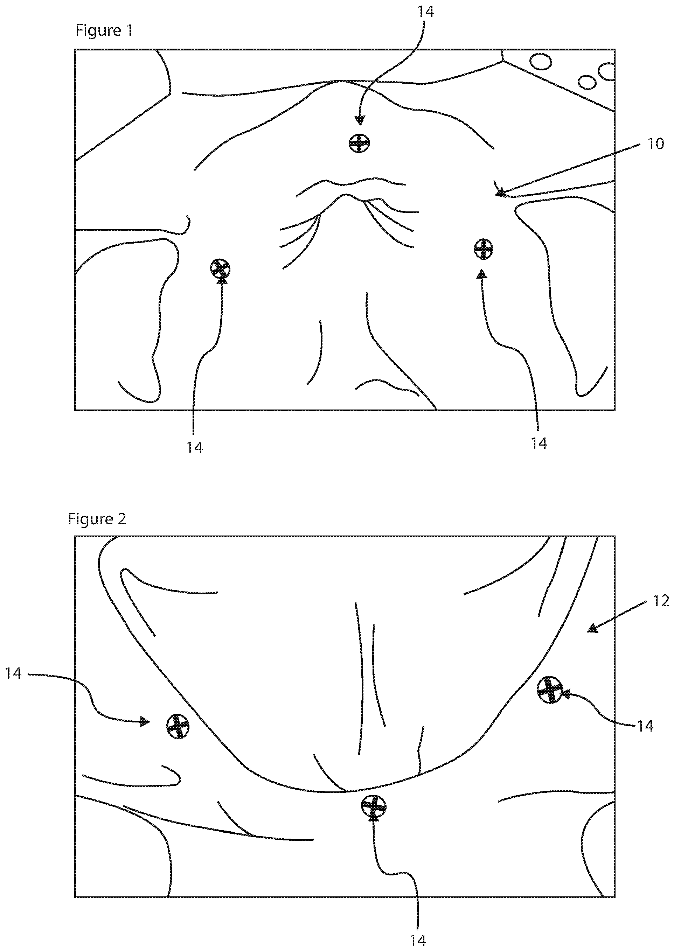

Figure 1 is a representation of a maxillary edentulous arch of a patient having a plurality of orthodontic screws inserted therein in a triangular formation;
Figure 2 is a representation of a mandibular edentulous arch of a patient having a plurality of orthodontic screws inserted therein in a triangular formation;
Figure 3 is a perspective representation of a dental implant to be inserted into a maxillary or mandibular edentulous arch of a patient;
Figure 4 is a cross-sectional image through a maxilla of a patient, indicating a viable dental-implant cavity which has been drilled, including an overlaid cross-sectional representation of the dental implant of Figure 3;
Figure 5 is a plan view of a first embodiment of a digital model of a dental implant drill-guide in accordance with the present invention;
Figure 6 is a plan view of a second embodiment of a digital model of a dental implant drill-guide in accordance with the present invention, interacting with a digital model of an edentulous arch of a patient;
Figure 7 is a plan view of a first embodiment of a dental implant drill-guide formed in accordance with the first aspect of the invention in combination with a dummy edentulous arch;
Figure 8 is an enlarged perspective representation of the dental implant drill-guide being inserted into the dummy edentulous arch using a two-part orthodontic screw;
Figure 9 is an exploded perspective representation of the two-part orthodontic screw shown in Figure 8, in isolation from the dental implant drill-guide;
Figure 10 is a plan view of a second embodiment of a dental implant drill-guide in accordance with the present invention; and
Figure 11 is a diagrammatic representation of a method of implanting one or more dental implants into a patient.
Referring firstly to Figures 1 and 2, maxillary and mandibular edentulous arches 10, 12 of a patient are shown, that is the toothless arches inside the mouth of a patient, prior to the installation of one or more dental implants to replace the patient's missing teeth.
A plurality of orthodontic screws 14 are inserted into the maxillary or mandibular edentulous arch 10, 12, and these will act as guides to the determination of dental implant positions. Preferably, three such orthodontic screws 14 are provided in a triangular formation so as to provide sufficient reference points in the maxillary or mandibular edentulous arch 10, 12 whilst creating minimal invasion into the patient's mouth.
It may be possible to pre-operatively image the maxillary or mandibular edentulous arch 10, 12 prior to insertion of the orthodontic screws 14 in order to not only determine an optimal position of each orthodontic screw 14, but also an optimal position of the dental implants to be installed. This may be achieved by radiographic imaging of the maxilla or mandible to determine a position of the osseous tissue.

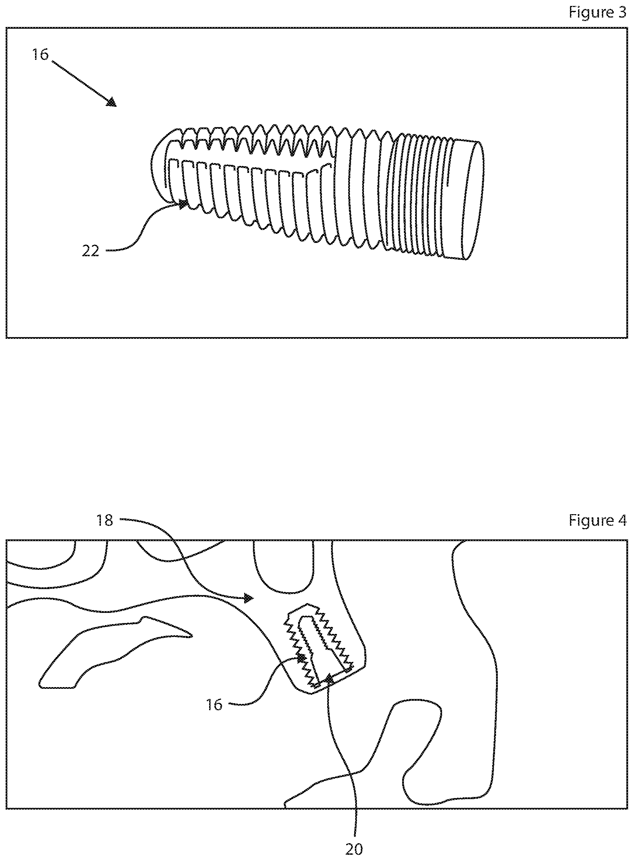

It is noted that the term orthodontic screw 14 is preferably intended to refer to a screw which can be inserted into the maxillary or mandibular edentulous arch 10, 12 of a patient, preferably so as to engage with the osseous tissue by no more than around 2 or 3 millimetres. This prevents the orthodontic screw 14 from integrating with the osseous tissue. On the other hand, a dental implant 16, such as that illustrated in Figure 3, is preferably designed to integrate with the osseous tissue 18 within a dental-implant cavity 20, such as that illustrated in Figure 4, and therefore the screw thread 22 of the dental implant 16 penetrates much more deeply into the osseous tissue 18 so as to form a more permanent bond in the patient's mouth.

Once an image of the maxillary or mandibular edentulous arch 10, 12 has been taken, the optimum positions of the orthodontic screws 14 and/or the dental-implant cavities 20 may be determined. Various factors may affect where the said optimum positions may be, for example, the intended positions of the dental implants 16, the underlying structure of the osseous tissue, the positions of nerve endings within the maxillary or mandibular edentulous arch 10, 12, and/or the angular positioning of the maxillary or mandibular edentulous arch 10, 12. This may typically be assessed by converting the image of the maxillary or mandibular edentulous arch 10, 12 into pre-operative imaging data which can be processed to determine the respective optimum positions of the orthodontic screws 14 and dental-implant cavities 20.

Once the dentist has inserted the plurality of orthodontic screws 14 into the maxillary or mandibular edentulous arch 10, 12 of the patient, it is possible to obtain mucosal impression data relating to the gingival mucosa of the patient. This may preferably be achieved by inserting an impression body into the mouth of the patient, which may be preferably formed from a mucostatic or mucocompressive material, about which the patient may clamp their jaws. This forms an imprint of the patient's gingival mucosa onto the impression body inclusive of an imprint of the heads of the orthodontic screws 14.

The orthodontic screws 14 therefore act to provide a rigid support for the impression body which is fixed relative to the osseous tissue 18 of the maxillary or mandibular edentulous arch 10, 12. Ordinarily, the gingival mucosa of an edentulous patient will compress during the creation of the impression, which would lead to an inaccurate impression relative to the osseous tissue. The impression may then be converted into mucosal impression data, preferably digital mucosal impression data, to provide three-dimensional information regarding the inside of the patient's mouth.

In addition to collating mucosal impression data, it is also possible to image the maxillary or mandibular edentulous arch 10, 12, preferably using a Cone Beam Computer Tomography (CBCT) scanning device to obtain a CBCT scan. This will yield imaging data relating to the maxillary or mandibular edentulous arch 10, 12 below the surface of the gingival mucosa in three dimensions, and will also image the orthodontic screws 14 to provide the necessary imaging data to calculate the drilling angles and positions in order to create the dental-implant cavities 20.

By combining the mucosal impression data with the imaging data of the maxillary or mandibular edentulous arch 10, 12, it is possible to determine dental implant position data for the insertion of one or more dental implants 16 into the edentulous maxillary or mandibular arch 10, 12 relative to the positions of the plurality of orthodontic screws 14. This may be achieved using a computer software matching program, for instance.

It will be appreciated that medical advances may progress such that the imaging of the patient's oral cavity and underlying maxilla and mandible can be achieved with a single scan, and therefore separate mucosal impression and CBCT scanning may not strictly be required. Furthermore, whilst the mucosal impression is presently performed prior to the CBCT scan, there is no strict requirement for this to be the case, and the order could be reversed.

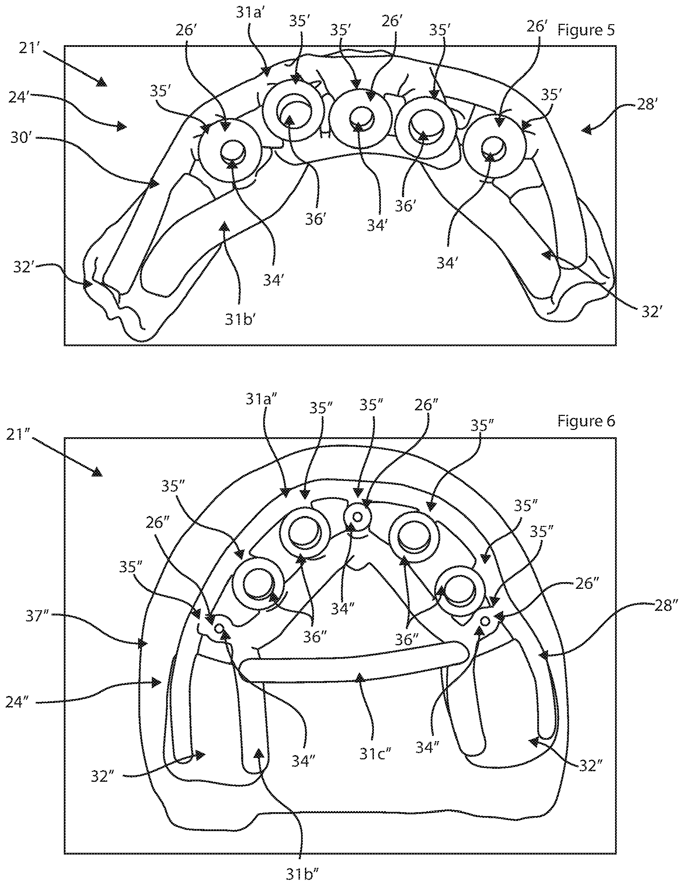

Once the dental implant position data has been created, it may be possible for dental implant drill-guide parameter data to be ascertained, preferably using computer software, which creates a digital model of a dental implant drill guide, such as the digital model 24' shown in Figure 5, having areas which are capable of guiding a dental drill into the mouth of the patient to create correctly positioned and oriented dental-implant cavities 20.

This digital model 21' is indicative of a physical dental implant drill-guide 24' having a plurality of orthodontic screw-seats 26' which are sized and shaped to be engagable with the said plurality of orthodontic screws 14. The dental implant drill-guide 24' is designed for at least the guiding of a drill in the oral cavity of a patient, and preferably also for the subsequent guidance of dental implants into the implant cavities formed by the drill.

The digital model 21' of the dental implant drill-guide 24' has a guide body 28' which is sized and shaped to be receivably insertable into the mouth of an edentulous patient at or adjacent to the maxillary or mandibular edentulous arch 10, 12. The representation of the guide body 28' here comprises an arched frame 30' which matches or substantially matches the curvature of the maxillary or mandibular edentulous arch 10, 12, which may be formed having anterior and posterior frame elements 31a, 31b. These anterior and posterior frame elements 31a', 31b' may be formed having unequal dimensions, which may assist with the comfortable seating of the dental implant drill-guide 24 in the patient's oral cavity. The arched frame 30' may terminate at each end in buccal abutment elements 32' which are seatable at or adjacent to the buccae of the edentulous patient. It will be appreciated, however, that the guide body 28' may be formed as a single contiguous element, into which apertures can be directly formed.

In the arched frame 30', a plurality of apertures may preferably be provided, which may be distinguishable as a set of first apertures 34' which are associable with the orthodontic screws 14 and a set of second apertures 36' which are associable with the dental implants 16. In the depicted embodiment of dental implant drill-guide 24', there are three first apertures 34', which may be held in place by bridging elements 35', and two second apertures 36', this being a digital model 21' of a dental implant drill-guide 24' for the installation of two dental implants 16 into the edentulous patient. Each of the second apertures 34', 36' may be also formed through bridging elements 35' preferably interposed between the anterior and posterior frame elements 31a', 31b'.

Here, the first apertures 34' are formed so as to each include an orthodontic screw-seat 26' which is engagable with, preferably the head of, an orthodontic screw 14 in the maxillary or mandibular edentulous arch 10, 12. This orthodontic screw-seat 26' may be formed as a flat surface of a bridging element 35' which is restable upon the heads of the orthodontic screws 14 when the dental implant drill-guide 24' is inserted into the mouth of a patient. However, the orthodontic screw-seat 26' may be formed so as to have a recess therein, or similar positive locator, which is engagable with the head of the orthodontic screw 14 to ensure a more secure positioning of the dental implant drill-guide 24' on the orthodontic screws 14.

The second apertures 36' are sized such that an appropriate dental drill-bit can pass through the second aperture 36' in a correct alignment so as to create the correctly positioned and oriented dental-implant cavities 20'. Typically, this will mean that the second apertures 36 have a greater width or diameter than the first apertures 34'. It will be appreciated that the second apertures 36' may not all be of a consistent size however; the second apertures 36' may be sized appropriately for a drill bit and/or dental implant 16 to pass therethrough, which may be different depending upon the size of dental-implant cavity 20 required.

The first apertures 34' may be sized so as to be sufficiently wide so as to receive a screw thread of an orthodontic screw 14 therethrough, but not the head of said orthodontic screw 14.

The bridging elements 35' may be specifically sized and aligned to readily guide a drilling direction. As can be seen in Figure 5, the angular alignment of the second apertures 36' may be skewed and/or non-parallel. This advantageously ensures that the most beneficial arrangement of the dental implants can be produced using a single dental implant drill-guide 24'.

A second embodiment of a digital model 21" of dental implant drill-guide 24" is shown in Figure 6. This embodiment is illustrated in conjunction with a digital model of a dummy edentulous arch 37". Identical or similar components of the first embodiment of the digital model 21' are indicated using identical or similar reference numerals, and further detailed description will be omitted for brevity.

In this embodiment, the digital model of the dummy edentulous arch 37" has been produced based on the mucosal impression data, whereas the digital model 21" of the dental implant drill guide 24" has been produced based on a combination of the mucosal impression data and imaging data.

The guide body 28" of the digital model 21" of dental implant guide 24" is similar to that of the first embodiment; there are anterior and posterior frame elements 31a", 31b" which have bridging elements 35" therebetween supporting a plurality of first and second apertures 34", 36". In this embodiment, the second apertures 36" are noticeably larger than the first apertures 34", such that a smaller clamping area for the orthodontic screw seats 26" is provided on the first apertures 34". Four second apertures 36" are provided here which are suitable for the implanting of four dental implants 20.

Instead of buccal abutment elements, the guide body 38" here terminates instead in gingival seats 32" which are suitable for seating onto the rear plateaus of the gingiva of a patient. A reinforcing frame element 31c" may also be provided which bridges the left and right sides of the posterior frame element 31b" to provide structural reinforcement of the dental implant guide 24".

The provision of the digital model of the dummy edentulous arch 37" and the digital model 21" of the dental implant drill-guide 24" allows for the exact positioning and form of the eventual physical dental implant drill-guide to be optimised for the specific patient.

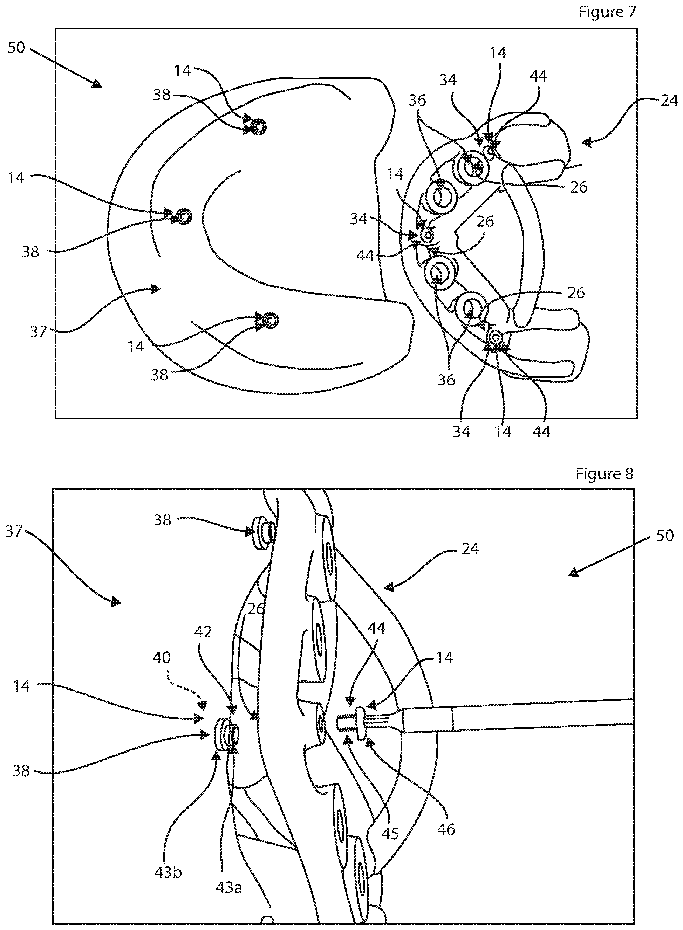

Once the digital model 21" of the dental implant drill-guide 24" has been satisfactorily completed, a physical dental implant drill guide 24 can be produced. Such a dental implant drill-guide 24 can be seen in Figure 7. The dental implant drill-guide 24 depicted has been formed as per the digital model 21" shown in Figure 6, and corresponding reference numerals will therefore be utilised to refer to corresponding components.

Typically, the dental implant drill-guide 24 will be formed from a non-toxic plastics material, and may be manufactured via moulding, extrusion, 3d printing, and/or any traditional CAD-CAM manufacturing process. Other manufacturing techniques will be apparent to the skilled person.

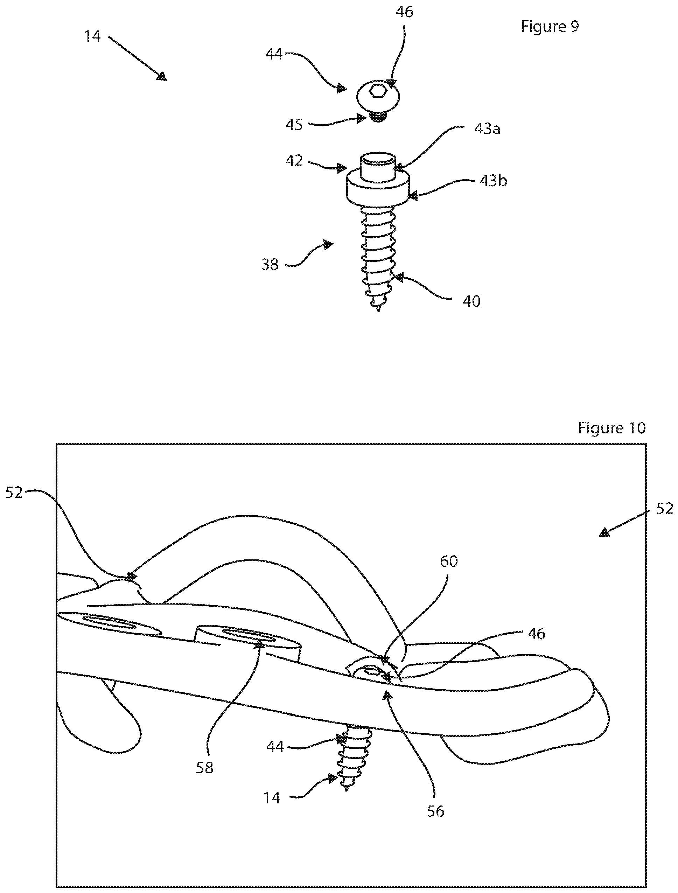

The dental implant drill-guide 24 is shown in conjunction with a dummy edentulous arch 37. This may be provided for training purposes only, in order to train dental surgeons in the use of the dental implant drill-guide 24.
To utilise the dental implant drill-guide 24 once manufactured, it is inserted into the oral cavity such that the orthodontic screw-seats 26 engage with the orthodontic screws 14 in position in the maxillary or mandibular edentulous arch 10, 12. In practice, the orthodontic screw in the maxillary or mandibular edentulous arch 10, 12 may only be one part of a two-part orthodontic screw 14, in this case, the apical part 38. The apical part 38 of the two-part orthodontic screw 14 has an apical screw thread 40, embedded into the dummy edentulous arch 37 in the Figures and indicated by the dashed line in Figure 8, and an apical head 42 which engages with the corresponding orthodontic screw-seat 26 when the dental implant drill-guide 24 is inserted into the oral cavity. This can be seen in Figures 7 and 8 of the drawings, and the two-part orthodontic screw is illustrated in detail in Figure 9; the apical part 38 of the two-part orthodontic screw 14 is inserted into the dummy edentulous arch 37. The apical head 42 is formed having a collar 43a which is at least in part receivable within a respective first aperture 34 of the dental implant drill-guide 24, and an apical flange 43b upon which the orthodontic screw-seat 26 is abuttably restable.
Once the dental implant drill-guide 24 is correctly seated on the apical parts 38 of the two-part orthodontic screws 14, preferably by seating on the apical flange 43b with the collar 43a entering into the first aperture 34, a coronal part 44 of the two-part orthodontic screw 14 can be inserted through each respective first aperture 34 of the dental implant drill-guide 24 to secure it in place. Each coronal part 44 has a coronal screw thread 45 which is receivable through apical head 42 of the apical part 38, preferably engaging with a corresponding screw thread within the collar 43a, and a coronal head 46 which abuttably engages with a top of the first aperture 34 with which it is engaged to apply a clamping force onto the dental implant drill-guide 24. This secures the dental implant drill-guide 24 relative to the osseous tissue of the maxillary or mandibular edentulous arch 10, 12. This process is best visualised from Figure 8.
This securing of the dental implant drill-guide 24 allows the dentist to safely drill into the maxillary or mandibular edentulous arch 10, 12 by guiding their drill bit through the respective second apertures 36 in order to form dental-implant cavities 20 in the osseous tissue of the maxillary or mandibular edentulous arch 10, 12. Once this has been performed, the two-part orthodontic screws 14 can be removed, releasing the dental implant drill-guide 24 from the maxillary or mandibular edentulous arch 10, 12. The dental implants 16 can then be inserted into their respective dental-implant cavities 20.

By using a dental implant drill-guide 24 which has been manufactured using the orthodontic screws 14 as reference points in rigid contact with the maxillary or mandibular edentulous arch 10, 12, the accuracy and reproducibility. with which dental-implant cavities 20 can be drilled is much improved. The dental-implant drill guide 24, in conjunction with the orthodontic screws 14, therefore represents an effective dental implant drill-guide system 50 which is capable of assisting with the insertion of dental implants into a patient's edentulous maxillary or mandibular arch 10, 12.

A second embodiment of a dental implant drill-guide can be seen in Figure 10, and is indicated globally at 52. The dental implant drill-guide 52 is substantially similar to that of the first embodiment, and further detailed description will be omitted, with the differences addressed.

The guide body 54 has first and second apertures 56, 58 as in the previously-described embodiment; however, the first apertures 56 are provided with an encapsulation portion 60 which holds the coronal part 44 of the two-part orthodontic screw 14 within the first aperture 56.

In the depicted embodiment, the encapsulation portion 60 is formed as a bridge which spans the top of the first aperture 56, retaining the coronal head 46 inside the first aperture 56. Preferably, the encapsulation portion 60 is formed so as to allow for limited longitudinal movement of the coronal part 44 within the first aperture 56, to better allow the dental surgeon to manipulate the coronal part 44 when installing the orthodontic screw 14.

Other encapsulation portions could be considered, for example, a perimeter lip, shoulder or stop around the upper circumference of the first aperture 56, which may abuttably engage with the coronal head 46. This would retain the coronal head 46 in position whilst providing unfettered access for the dental surgeon to the drive of the coronal head 44 with a screwdriver or similar implement.

Furthermore, the first aperture 56 may be provided so as to have a positive engagement means with the coronal part 44 of the two-part orthodontic screw 14. For example, one or more teeth or grips may be provided which inhibit movement of the coronal part 44 in a direction away from the apical part 38, that is, only permitting movement of the coronal part 44 in a direction towards securing of the two-part orthodontic screw 14 together.

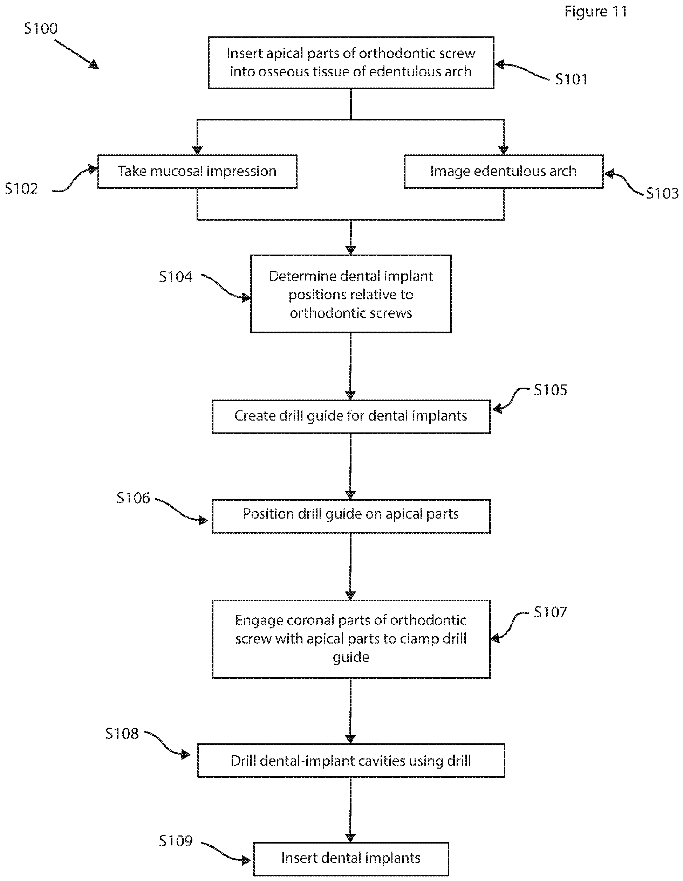

The entire process can therefore be summarised as follows, and as indicated in Figure 11 globally at S100. A plurality of apical parts 38 of two-part orthodontic screws 14 can be inserted, at step S101, into an edentulous maxillary or mandibular arch 10, 12 of a patient, such that the two-part orthodontic screws 14 engage with the osseous tissue of the edentulous maxillary or mandibular arch 10, 12. A mucosal impression of the edentulous maxillary or mandibular arch 10, 12 can then be obtained, at step S102, and the maxilla or mandible of the patient inclusive of the plurality of two-part orthodontic screws can be imaged, at step S 103, to form a maxillary or mandibular image.

One or more dental implant positions can be determined, at step S104, from the maxillary or mandibular image and the mucosal impression for the insertion of dental implants 16 into the edentulous maxillary or mandibular arch 10, 12 relative to the positions of the plurality of two-part orthodontic screws 14, which in turn allows a dental implant drill-guide 24 to be created, at step S105, based on the one or more dental implant positions and the positions of the plurality of two-part orthodontic screws 14.

The manufactured dental drill-guide 24 can then be positioned, at step S106, on the apical parts 38 of the two-part orthodontic screws 14, the dental implant drill-guide 24 being fixed, at step S107, in position by engaging the coronal parts 44 of the two-part orthodontic screws 14 with the apical parts 38 of the two-part orthodontic screws 14 so as to clamp the dental implant drill-guide 24.

One or more dental-implant cavities 20 can then be drilled, at step S108, in the edentulous maxillary or mandibular arch 10, 12 using the dental implant drill-guide 24, and a drill cylinder or collar may be used to accurately control the angle and depth of penetration of the drill into the patient's osseous tissue 18. Such a drill cylinder or collar may be receivable within the second apertures 36, thereby preventing or limiting damage to the dental implant drill-guide 24 by the drill. A dental implant 16 is then inserted, at step S109, into each dental-implant cavity 20, preferably by using a key or similar engagement tool to screw or urge the dental implant 16 into position.

In a preferred embodiment, the dental implant 16 may be inserted whilst the dental implant drill-guide 24 is still secured in position in the patient's oral cavity, since the alignments of the second apertures 36 are such that the dental implants 16 will be readily guidable into their respective dental-implant cavities 20. On the other hand, the dental implant drill-guide 24 may block or impede the insertion of the dental implants 16, and therefore it may be prudent to first remove the dental implant drill-guide 24 prior to insertion of the dental implants 16.

Whilst dental implants are suggested as being the desired items to install into the patient's mouth, it will be appreciated that drill-guide geometry formed in accordance with the present invention could be used in the context of any oral drilling application, either for edentulous patients or partially edentulous patients.

Whilst it is suggested that the same set of orthodontic screws may be used both to reference the position of the osseous tissue in the patient and then to secure the dental implant drill guide in position, it may be possible to use a first set of orthodontic screws in the referencing stage, and then only utilise the two-part screws at a later juncture.

It is therefore possible to provide a method of more accurately and reproducibly drilling dental implant cavities into the edentulous maxillary or mandibular arch of a patient by manufacturing a dental implant drill-guide which is bespoke to the patient. This can be achieved by imaging the edentulous maxillary or mandibular arch of the patient following the insertion of a plurality of orthodontic screws therein. This means that the images taken of the mandible or maxilla have a fixed reference thereon, and from the positions of the orthodontic screws, an appropriately-shaped drill guide can be produced which is seatable upon the orthodontic screws.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

## Claims

1. A dental implant drill-guide system (50) comprising:
a plurality of two-part orthodontic screws (14), a coronal part (44) of each two-part orthodontic screw (14) having a screw-thread which is receivable within an apical part (38) of the two-part orthodontic screw (14); and
a dental implant drill-guide (24), the dental implant drill-guide (24) having
a guide body (54) which is complementarily-shaped to an edentulous maxillary or mandibular arch (10, 12) of a patient,
a plurality of first apertures (34; 56) through the guide body (54) each having an orthodontic screw-seat (26) associated therewith which is engagable with the apical part (38) of one said two-part orthodontic screw (14), and
at least one second aperture (36; 58) which is oriented and sized to form a drill-guide through which a dental drill for drilling a dental-implant cavity (20) in the edentulous maxillary or mandibular arch (10, 12) can pass; and
wherein the apical part (38) of the two-part orthodontic screws (14) includes an apical flange (43b) and a collar (43a) extending upwardly from the apical flange (43b), each two-part orthodontic screw-seat (26) being formed as a flat surface on a base of the guide body (34; 54) at a respective first aperture (56), the coronal part (44) of the two-part orthodontic screws (14) abuttably engaging with a top of said first aperture (34; 54) to apply a clamping force onto the dental implant drill-guide (24) when threaded into the apical part (38) of the two-part orthodontic screws (14).

2. A dental implant drill-guide system (50) as claimed in claim 1, wherein the or each second aperture (36; 58) is larger than the or each first aperture (34; 56) of the dental implant drill-guide (24).

3. A dental implant drill-guide system (50) as claimed in any one of the preceding claims, wherein the guide body (54) comprises an anterior frame element (31a), a posterior frame element (31b), and at least one bridging elements (35), the first and second apertures (34, 36; 56, 58) being located within the or each bridging element (35).

4. A dental implant drill-guide system (50) as claimed in any one of the preceding claims, wherein the guide body (54) comprises at least one buccal abutment element (32).

5. A dental implant drill-guide system (50) as claimed in any one of the preceding claims, wherein the coronal part (44) of each two-part orthodontic screw (14) is held captive within each respective first aperture (34; 56).

6. A dental implant drill-guide system (50) as claimed in any one of the preceding claims, wherein the apical part (38) of the two-part orthodontic screw (14) is a non-integrating orthodontic screw, the apical part (38) of the two-part orthodontic screw (14) acts as a positional reference for the dental implant drill-guide (24).

## Patentansprüche

1. Führungssystem (50) für Zahnimplantatbohrer, umfassend:
eine Vielzahl zweiteiliger orthodontischer Schrauben (14), wobei ein koronaler Teil (44) jeder zweiteiligen orthodontischen Schraube (14) ein Schraubgewinde aufweist, das in einem apikalen Teil (38) der zweiteiligen orthodontischen Schraube (14) aufnehmbar ist; und
eine Zahnimplantatbohrerführung (24), wobei die Zahnimplantatbohrerführung (24) Folgendes aufweist:
einen Führungskörper (54), der komplementär zu einem zahnlosen maxillaren oder mandibularen Bogen (10, 12) eines Patienten geformt ist,
eine Vielzahl erster Öffnungen (34; 56) durch den Führungskörper (54), die jeweils einen zugeordneten orthodontischen Schraubensitz (26) aufweisen, der mit dem apikalen Teil (38) einer der zweiteiligen orthodontischen Schrauben (14) in Eingriff treten kann, und
wenigstens eine zweite Öffnung (36; 58), die dazu ausgerichtet und dimensioniert ist, eine Bohrungsführung zu bilden, durch die ein Zahnbohrer zum Bohren einer Zahnimplantatkavität (20) in dem zahnlosen maxillaren oder mandibularen Bogen (10, 12) treten kann; und
wobei der apikale Teil (38) der zweiteiligen orthodontischen Schrauben (14) einen apikalen Flansch (43b) und einen Hals (43a) beinhaltet, der sich von dem apikalen Flansch (43b) nach oben erstreckt, wobei jeder zweiteilige orthodontische Schraubensitz (26) als eine flache Oberfläche auf einer Basis des Führungskörper (34; 54) an einer jeweiligen ersten Öffnung (56) gebildet ist, wobei der koronale Teil (44) der zweiteiligen orthodontischen Schrauben (14) mit einer Oberseite der ersten Öffnung (34; 54) in Anlageeingriff tritt, um beim Einschrauben in den apikalen Teil (38) der zweiteiligen orthodontischen Schrauben (14) eine Klemmkraft auf die Zahnimplantatbohrerführung (24) auszuüben.

2. Führungssystem (50) für Zahnimplantatbohrer nach Anspruch 1, wobei die oder jede zweite Öffnung (36; 58) größer als die oder jede erste Öffnung (34; 56) der Zahnimplantatbohrerführung (24) ist.

3. Führungssystem (50) für Zahnimplantatbohrer nach einem der vorangehenden Ansprüche, wobei der Führungskörper (54) ein vorderes Rahmenelement (31a), ein hinteres Rahmenelement (31b) und wenigstens ein Brückenelement (35) umfasst, wobei die erste und die zweite Öffnung (34, 36; 56, 58) in dem oder jedem Brückenelement (35) angeordnet sind.

4. Führungssystem (50) für Zahnimplantatbohrer nach einem der vorangehenden Ansprüche, wobei der Führungskörper (54) wenigstens ein bukkales Anlageelement (32) umfasst.

5. Führungssystem (50) für Zahnimplantatbohrer nach einem der vorangehenden Ansprüche, wobei der koronale Teil (44) jeder zweiteiligen orthodontischen Schraube (14) in jeder jeweiligen ersten Öffnung (34; 56) festgehalten wird.

6. Führungssystem (50) für Zahnimplantatbohrer nach einem der vorangehenden Ansprüche, wobei der apikale Teil (38) der zweiteiligen orthodontischen Schraube (14) eine nicht integrierende orthodontische Schraube ist, wobei der apikale Teil (38) der zweiteiligen orthodontischen Schraube (14) als eine Positionsreferenz für die Zahnimplantatbohrerführung (24) dient.

## Revendications

1. Système de guide de foret d'implant dentaire (50) comprenant :
une pluralité de vis orthodontiques en deux parties (14), une partie coronale (44) de chaque vis orthodontique en deux parties (14) présentant un filetage qui est recevable à l'intérieur d'une partie apicale (38) de la vis orthodontique en deux parties (14) ; et
un guide de foret d'implant dentaire (24), le guide de foret d'implant dentaire (24) présentant
un corps de guidage (54) qui est façonné de manière complémentaire à un maxillaire édenté ou à une arche mandibulaire (10, 12) d'un patient,
une pluralité de premières ouvertures (34 ; 56) à travers le corps de guidage (54) présentant chacune un siège de vis orthodontique (26) associé à celleci qui peut entrer en prise avec la partie apicale (38) de l'une desdites vis orthodontiques en deux parties (14) et
au moins une seconde ouverture (36 ; 58), qui est orientée et dimensionnée pour former un guide de foret à travers lequel peut passer un foret dentaire destiné à forer une cavité d'implant dentaire (20) dans le maxillaire édenté ou l'arche mandibulaire (10, 12) ; et
dans lequel la partie apicale (38) des vis orthodontiques en deux parties (14) comporte un collet apical (43b) et une bague (43a) s'étendant vers le haut depuis le collet apical (43b), chaque siège de vis orthodontique en deux parties (26) étant formé comme une surface plane sur une base du corps de guidage (34 ; 54) au niveau d'une première ouverture respective (56), la partie coronale (44) des vis orthodontiques en deux parties (14) entrant en prise en butée avec une partie supérieure de ladite première ouverture (34 ; 54) pour appliquer une force de serrage sur le guide de foret d'implant dentaire (24) lorsqu'il est vissé dans la partie apicale (38) des vis orthodontiques en deux parties (14).

2. Système de guide de foret d'implant dentaire (50) selon la revendication 1, dans lequel la ou chaque seconde ouverture (36 ; 58) est plus grande que la ou chaque première ouverture (34 ; 56) du guide de foret d'implant dentaire (24).

3. Système de guide de foret d'implant dentaire (50) selon l'une quelconque des revendications précédentes, dans lequel le corps de guidage (54) comprend un élément de cadre antérieur (31a), un élément de cadre postérieur (31b) et au moins un élément de pontage (35), les première et seconde ouvertures (34, 36 ; 56, 58) étant situées à l'intérieur de l'élément ou de chaque élément de pontage (35).

4. Système de guide de foret d'implant dentaire (50) selon l'une quelconque des revendications précédentes, dans lequel le corps de guidage (54) comprend au moins un élément d'appui buccal (32).

5. Système de guide de foret d'implant dentaire (50) selon l'une quelconque des revendications précédentes, dans lequel la partie coronale (44) de chaque vis orthodontique en deux parties (14) est maintenue captive à l'intérieur de chaque première ouverture respective (34 ; 56).

6. Système de guide de foret d'implant dentaire (50) selon l'une quelconque des revendications précédentes, dans lequel la partie apicale (38) de la vis orthodontique en deux parties (14) est une vis orthodontique non intégrante, la partie apicale (38) de la vis orthodontique en deux parties (14) agit comme une référence de position destinée au guide de foret d'implant dentaire (24).
